# FASCICULE DE BREVET EUROPEEN

(11) **EP 3 447 492 B1**
(45) Date de publication et mention de la délivrance du brevet: **29.01.2025**
(21) Numéro de dépôt: 18191274.2
(22) Date de dépôt: 26.11.2013
(51) Int. Cl.: G01N 33/543, G01N 27/74, B01L 3/00, H01F 1/00

(54) **PROCEDE DE CAPTURE ET DE DETECTION SANS LAVAGE D'UNE MOLECULE DANS UN ECHANTILLON**
VERFAHREN ZUM EINFANGEN UND ENTDECKEN OHNE WASCHEN EINES MOLEKÜLS IN EINER PROBE
METHOD FOR CAPTURING AND DETECTION WITHOUT WASHING OF A MOLECULE IN A SAMPLE

(30) Priorité: 17.01.2013 FR 1350421
(43) Date de publication de la demande: 27.02.2019
(62) Demande divisionnaire de: 13795765.0
(73) Titulaire: CENTRE NATIONAL DE LA RECHERCHE SCIENTIFIQUE, 75016 Paris (FR); Institut Polytechnique de Grenoble, 38000 Grenoble (FR)
(72) Inventeur: BLAIRE, Guillaume, 74300 Magland (FR); ZANINI, Luiz Fernando, Prague 160 00 (CZ); REYNE, Gilbert, 13008 Marseille (FR); DEMPSEY, Nora, 38000 Grenoble (FR); WEIDENHAUPT, Marianne, 38134 St Joseph de Rivière (FR); BRUCKERT, Franz, 38100 Grenoble (FR); CUGAT, Orphée, 38320 Poisat (FR)
(74) Mandataire: Hautier IP

(56) Documents cités:
- WO-A1-2007/125129
- DAVID ISSADORE ET AL: "Self-assembled magnetic filter for highly efficient immunomagnetic separation", LAB ON A CHIP, vol. 11, no. 1, 1 January 2011 (2011-01-01), pages 147, XP055049322, ISSN: 1473-0197, DOI: 10.1039/c0lc00149j
- LUIZ FERNANDO ZANINI: "Bio-Mag-MEMS autonomes basés sur des aimants permanents", THÈSE POUR OBTENIR LE GRADE DE DOCTEUR DE L'UNIVERSITÉ DE GREN,, 18 February 2013 (2013-02-18), pages 1 - 166, XP007922459, Retrieved from the Internet <URL:http://www.theses.fr/2013GRENT004/abes>
- GUILLAUME BLAIRE ET AL: "Hybrid Bio-Mag-MEMS combining magnetophoresis and dielectrophoresis", THE EUROPEAN PHYSICAL JOURNAL B, vol. 86, no. 4, 1 April 2013 (2013-04-01), XP055094026, ISSN: 1434-6028, DOI: 10.1140/epjb/e2013-30679-1
- OSMAN O ET AL: "Monitoring the endocytosis of magnetic nanoparticles by cells using permanent micro-flux sources", BIOMEDICAL MICRODEVICES, KLUWER ACADEMIC PUBLISHERS, BO, vol. 14, no. 5, 7 July 2012 (2012-07-07), pages 947 - 954, XP035113960, ISSN: 1572-8781, DOI: 10.1007/S10544-012-9673-4

## Description

### DOMAINE DE L'INVENTION

La présente invention concerne un procédé de capture et un procédé de détection, notamment par immunodosage (« immunoassay » selon la terminologie anglo-saxonne) d'une molécule dans un échantillon, ladite molécule à capturer ou à détecter étant typiquement un antigène ou un anticorps. Ci-dessous est également décrit, mais ne faisant pas partie de l'invention revendiquée, un kit de capture d'une molécule adapté pour la mise en oeuvre desdits procédés.

### ARRIERE PLAN DE L'INVENTION

Le test ELISA (acronyme du terme anglo-saxon « Enzyme-Linked Immuno Sorbent Assay ») est utilisé couramment pour diagnostiquer de manière quantitative des marqueurs moléculaires (antigènes ou anticorps) présents dans des fluides, des biopsies, des cultures ou tout autre échantillon.

Ce test est en particulier utilisé pour diagnostiquer des infections (VIH, HPV, etc.), l'état du système cardio-vasculaire et immunitaire (Troponine C, anticorps spécifiques, etc.), la présence et le développement de cancers (PSA, CEA, etc.).

Cette technique, qui est à l'heure actuelle la plus robuste et la plus répandue des méthodes de diagnostic, possède cependant des inconvénients, à savoir sa complexité, l'utilisation d'automates coûteux et sa durée qui peut atteindre quelques heures.

Le test ELISA est une technique d'immunodosage en phase hétérogène, c'est-à-dire qu'elle nécessite un support solide (typiquement, une plaque de titration comprenant une pluralité de puits) auquel on fixe au préalable une molécule adaptée pour capturer la molécule à doser.

Une fois que la molécule d'intérêt a été capturée sur ledit support, un lavage permet de retirer le reste de l'échantillon et de procéder à l'étape de détection et de quantification de ladite molécule.

Par exemple, dans le cas du test ELISA dit « sandwich », qui permet de doser un antigène dans une solution, on recouvre la surface du support d'une quantité déterminée d'un anticorps dit de capture, ledit anticorps étant adapté pour se lier à l'antigène recherché.

Puis on applique sur le support la solution susceptible de contenir ledit antigène ; ledit antigène se lie alors à l'anticorps de capture situé sur la surface du support.

Ensuite, on effectue un lavage du support de sorte à retirer l'antigène non lié restant éventuellement dans la solution.

On dépose alors sur le support une solution contenant un anticorps dit anticorps de détection, qui est adapté pour se lier à l'antigène fixé sur le support et qui est par ailleurs couplé à une enzyme.

On met en œuvre une nouvelle étape de lavage, de sorte à conserver sur le support l'antigène lié à l'anticorps de détection, ledit anticorps étant lui-même couplé à l'enzyme.

Enfin, pour la détection et la quantification de l'antigène, on dépose sur le support un substrat qui est converti par l'enzyme en un signal détectable (par exemple une couleur ou spectroscopiquement, c'est-à-dire par émission de fluorescence) représentatif de la liaison entre l'antigène et l'anticorps de détection.

Ledit signal peut être observé à l'œil nu ou au moyen d'un instrument, tel qu'un spectrophotomètre.

Le test ELISA comprend différentes implémentations mais elles comprennent toutes de multiples étapes de greffage et d'incubation séparées par des étapes de lavage.

La durée importante du test ELISA est essentiellement due à la succession des différentes étapes.

Des variantes de ce test mettant en œuvre des microbilles magnétiques ont déjà été décrites.

L'article de D. Issadore et al, Self-assembled magnetic filter for highly efficient immunomagnetic separation, Lab Chip, 2011, 11, 147 décrit ainsi un procédé de capture d'une molécule dans un échantillon en faisant circuler ledit échantillon dans un microcanal fluidique agencé au-dessous d'une matrice de polydiméthylsiloxane (PDMS) dans laquelle des grains magnétiques de NdFeB ont été immobilisés.

De par le procédé de fabrication mis en œuvre pour ledit support, les grains sont répartis de manière aléatoire dans l'épaisseur de la matrice et leur orientation magnétique est aléatoire.

Chaque grain génère un gradient de champ magnétique qui est susceptible d'attirer des particules magnétiques d'un diamètre de 1 µm couplées aux molécules à détecter pour former des complexes.

Cependant, un tel gradient de champ magnétique est d'autant moins efficace que la taille des particules à capturer est faible, de sorte que ce dispositif est moins performant pour la capture de particules magnétiques de dimensions inférieures à 1 µm, par exemple de l'ordre de 500 nm ou moins, et notamment des nanoparticules.

D'autre part, la capture des particules magnétiques est relativement lente.

Cette étape de capture est suivie d'un lavage de la surface de la matrice de PDMS puis d'une détection par fluorescence des complexes immobilisés sur ladite surface. Guillaume Blaire et al, 2013 (Eur Phys J B, 86(4), 165, 1-6) décrit un procédé de capture et de détection par fluorescence d'une molécule dans un échantillon, dans lequel ladite molécule est mélangée à des nanoparticules magnétiques, les complexes sont immobilisés sur un microcanal. Les micro-sources de champ magnétique sont ordonnées et la détection de complexes est réalisée par fluorescence. Le procédé comprend une étape de lavage et utilise une concentration de nanoparticules inférieure à 10⁶ particules/ml.

### BREVE DESCRIPTION DE L'INVENTION

Un but de l'invention est de proposer un procédé de détection d'une molécule telle qu'antigène ou d'un anticorps nécessitant un nombre limité d'étapes, voire une seule étape, et qui soit sensiblement plus court que les tests connus.

Ce procédé doit par ailleurs être compatible avec l'instrumentation déjà utilisée par les biologistes, telle que les plaques de titration, les fluorimètres, les pipettes multicanaux, de sorte à pourvoir être directement mis en oeuvre dans des laboratoires de recherche et d'analyse.

Selon la description, mais ne faisant pas partie de l'invention revendiquée, il est proposé un procédé de capture d'une molécule dans un échantillon, comprenant les étapes suivantes :
a) le mélange dudit échantillon avec des particules magnétiques, chacune desdites particules étant couplée avec un élément apte à se lier sélectivement à ladite molécule à capturer, de sorte à former au moins un complexe comprenant une particule magnétique
b) l'immobilisation dudit au moins un complexe sur un support comprenant des microsources de champ magnétique ordonnées.

Conformément à l'invention, il est proposé un procédé de capture d'une molécule dans un échantillon, comprenant les étapes suivantes :
a) le mélange dudit échantillon avec des particules magnétiques, chacune desdites particules étant couplée avec un élément apte à se lier sélectivement à ladite molécule à capturer, de sorte à former au moins un complexe comprenant une particule magnétique, ledit élément et ladite molécule liée audit élément, ledit élément et ladite molécule liée audit élément, ces particules magnétiques présentant un diamètre compris entre 5 nm et 500 nm,
b) l'immobilisation dudit au moins un complexe sur un support comprenant des microsources de champ magnétique ordonnées,
c) la détection par fluorescence de la molécule immobilisée,

où l'organisation spatiale des micros sources de champ magnétique ordonnées définit des zones de gradient de champ magnétique fort et des zones de gradient de champ magnétique faible, en ce que
l'étape de détection est mise en oeuvre directement après l'étape d'immobilisation, sans étape intermédiaire de lavage du support, par la détermination de la densité de fluorescence à la surface des micros sources de champ magnétique ordonnées dans les zones de gradient de champ magnétique fort, définissant un signal spécifique, et dans les zones de gradient de champ magnétique faible, définissant un signal non spécifique,
où ledit support est un microcanal comprenant dans au moins une de ses parois un réseau de micro-sources magnétiques agencées de manière ordonnée,
et où la concentration de particules magnétiques dans le mélange obtenu à l'issue de l'étape a) est supérieure à 10⁶ particules/ml.

Par « ordonnées », on entend que les micro-sources de champ magnétique sont réparties au voisinage de la surface du support destinée à être en contact avec l'échantillon selon un motif déterminé et qu'elles présentent en outre une orientation magnétique déterminée. Un tel ordonnancement exclut de fait une répartition et/ou une orientation aléatoire des micro-sources de champ magnétique.

Selon un mode de réalisation de la description, mais ne faisant pas partie de l'invention revendiquée, le procédé comprend une étape de lavage dudit support pour retirer ledit échantillon, les complexes étant retenus sur le support par les micro-sources de champ magnétique.

Selon invention, la détection des complexes est mise en oeuvre au niveau des micro-sources de champ magnétiques ordonnées, sans lavage préalable du support.

Par molécule, on entend dans le présent texte une protéine, notamment un anticorps, un acide nucléique (ADN ou ARN), un virus, une bactérie, un antigène, une cellule et de manière générale tout objet biologique.

Selon un mode de réalisation de la description, mais ne faisant pas partie de l'invention revendiquée, les particules magnétiques présentent un diamètre compris entre 5 nm et 1 µm.

Selon l'invention, les particules magnétiques présentent un diamètre compris entre 5 nm et 500 nm.

De préférence, les particules magnétiques présentent un diamètre compris entre 5 et 50 nm.

Selon un mode de réalisation, le support comprend une matrice non magnétique renfermant une pluralité d'agglomérats tridimensionnels ordonnés de micro ou nanoparticules aimantées d'un matériau magnétique dur ou doux, lesdits agglomérats formant les micro-sources de champ magnétique.

Selon un autre mode de réalisation, le support comprend une matrice non magnétique renfermant une pluralité de micro-bobines magnétiques ordonnées selon un motif déterminé par rapport à la surface du support.

Ladite matrice non magnétique peut être flexible.

En outre, ladite matrice non magnétique peut être en un matériau translucide ou transparent.

Selon une forme d'exécution de l'invention, la molécule à capturer est un antigène et l'élément apte à se lier à ladite molécule est un anticorps récepteur dudit antigène.

Eventuellement, on ajoute audit mélange un anticorps dit de détection portant un marqueur fluorescent, luminescent ou colorimétrique, ledit anticorps étant apte à se lier à l'antigène lié à l'anticorps couplé à la particule magnétique. L'anticorps de détection peut également être marqué par une enzyme catalysant une réaction redox permettant une détection électrochimique.

Selon une autre forme d'exécution de l'invention, la molécule à capturer est un anticorps et l'élément apte à se lier à ladite molécule est un antigène reconnu par ledit anticorps.

Selon un mode de réalisation de la description, mais ne faisant pas partie de l'invention revendiquée, le support est une plaque de titration comprenant une pluralité de puits, les micro-sources de champ magnétique étant agencées dans une paroi de chacun desdits puits, le mélange étant déposé dans au moins un desdits puits.

Selon un autre mode de réalisation de la description, mais ne faisant pas partie de l'invention revendiquée, le support est une plaque plane comprenant une pluralité de micro-sources de champ magnétique, le mélange étant déposé sous la forme d'au moins une goutte sur ledit support.

Selon l'invention, le support est un canal microfluidique dont au moins une des parois comprend des micro-sources de champ magnétique.

Un autre objet de l'invention concerne un procédé de détection et, le cas échéant, de quantification d'une molécule, dans lequel on capture ladite molécule sur un support en mettant en œuvre le procédé de capture décrit plus haut, puis l'on met en oeuvre une étape de détection de ladite molécule.

Selon la description, mais ne faisant pas partie de l'invention revendiquée, la détection de la molécule capturée sur le support peut être réalisée par luminescence, colorimétrie, électrochimie et/ou radiométrie.

Selon l'invention, la détection de la molécule capturée sur le support est réalisée par fluorescence.

Un premier avantage de ce procédé est la réduction significative du nombre d'étapes préalables à la détection et, le cas échéant, à la quantification (seules une étape de mélange et, éventuellement, une étape de lavage étant nécessaires), par rapport aux tests d'immunodosage connus (le test ELISA sandwich standard comprenant au minimum six étapes).

En effet, l'attraction des particules magnétiques par les micro-sources de champ magnétique permet d'une part d'extraire rapidement les objets associés aux dites particules et assure d'autre part un lavage plus efficace (dans les cas de la description, mais ne faisant pas partie de l'invention revendiquée, où un tel lavage est mis en œuvre), les forces d'interaction magnétiques retenant fermement les complexes immobilisés que l'on souhaite conserver.

Par ailleurs, ce procédé offre une alternative à l'ensemble des implémentations possibles du test ELISA, et peut être mis en œuvre pour réaliser des tests de type « sandwich » mais aussi des tests directs ou des tests par compétition en choisissant les antigènes et anticorps adaptés.

Ce procédé est également adaptable pour réaliser des tests d'immunoprécipitation.

Un autre avantage de ce procédé est sa grande simplicité de mise en œuvre et son faible coût, associé à l'absence de nécessité de source d'énergie exteme pour la capture des complexes et le lavage.

Dans la mesure où l'on peut s'affranchir de l'étape de lavage préalable à la détection, le procédé peut ne comprendre qu'une unique étape de mélange au contact du support comprenant les micro-sources de champ magnétique ordonnées.

Du fait de cette simplicité, le procédé pourrait être mis en œuvre dans un dispositif de test intégré de type « point-of-care ».

Un autre objet de la description, mais ne faisant pas partie de l'invention revendiquée, concerne un kit de capture d'une molécule, comprenant un support comprenant des micro-sources de champ magnétique ordonnées et une pluralité de particules magnétiques.

Lesdites particules magnétiques présentent une surface fonctionnalisée adaptée pour le couplage d'un élément apte à se lier sélectivement à ladite molécule.

Selon un mode de réalisation, mais ne faisant pas partie de l'invention revendiquée, le support est une plaque de titration comprenant une pluralité de puits, les micro-sources de champ magnétique étant agencées dans au moins une paroi de chaque puits.

Selon un mode de réalisation, mais ne faisant pas partie de l'invention revendiquée, le support est une plaque plane à la surface de laquelle sont agencées les micro-sources de champ magnétique.

Selon un mode de réalisation, le support est un microcanal fluidique, les microsources de champ magnétique étant agencées à la surface d'au moins une des parois dudit microcanal.

Un autre avantage de ce procédé est de permettre une détection sans lavage de la molécule capturée, grâce à la connaissance par l'expérimentateur des zones où les particules magnétiques sont capturées. Le signal spécifique est obtenu en soustrayant au signal total un signal correspondant à la capture non-spécifique des molécules.

Selon un mode de réalisation, mais ne faisant pas partie de l'invention revendiquée, les molécules sont détectées par fluorescence, luminescence ou colorimétrie grâce à un anticorps de détection. Une image de fluorescence, de luminescence ou d'absorbance de la surface portant les micro-aimants permet de déterminer le signal spécifique et non-spécifique. Le signal total correspond à la présence de molécules au niveau des zones de fort gradient de champ magnétique, le signal non-spécifique correspond à la présence de molécules dans les zones de faible gradients de champ magnétique.

Selon un autre mode de réalisation, mais ne faisant pas partie de l'invention revendiquée, les molécules sont détectées par électrochimie grâce à un anticorps de détection couplé à une enzyme ayant des propriétés redox. Des électrodes sont disposées sur la surface du support de façon à permettre la mesure du signal total et non-spécifique. Le signal total correspond à des électrodes disposées au niveau des micro-sources de champ magnétique ordonnées, le signal non-spécifique correspond à des électrodes disposées au niveau de zones dépourvues de micro-sources de champ magnétique.

### BREVE DESCRIPTION DES DESSINS

D'autres caractéristiques et avantages de l'invention ressortiront de la description détaillée qui va suivre, en référence aux dessins annexés sur lesquels :
- les figures 1A à 1C sont des vues schématiques des étapes de mise en oeuvre du procédé de détection selon un mode de réalisation, ne faisant pas partie de l'invention revendiquée mais utile pour sa compréhension,
- la figure 2, ne faisant pas partie de l'invention revendiquée mais utile pour sa compréhension, illustre de manière schématique une surface magnétiquement structurée de sorte à former des micro-sources de champ magnétique,
- les figures 3A à 3D, ne faisant pas partie de l'invention revendiquée mais utile pour sa compréhension, illustrent de manière schématique les différentes étapes du procédé d'empreinte thermo-magnétique permettant de réaliser un support comprenant des micro-sources de champ magnétique,
- les figures 4A à 4D, ne faisant pas partie de l'invention revendiquée mais utile pour sa compréhension, illustrent de manière schématique les différentes étapes du procédé dit « Micro Magnetic Imprinting »,
- la figure 5, ne faisant pas partie de l'invention revendiquée mais utile pour sa compréhension, illustre une forme d'exécution de l'invention dans laquelle le mélange est déposé sur un support plan sous la forme d'une goutte,
- la figure 6, ne faisant pas partie de l'invention revendiquée mais utile pour sa compréhension, présente une comparaison des pourcentages de billes capturées sur un support plan et dans un puits de titration en fonction du temps,
- les figures 7A à 7E illustrent un procédé de fabrication d'un microcanal fluidique comprenant des micro-sources de champ magnétique permettant la mise en oeuvre du procédé selon l'invention,
- la figure 8 est une courbe du signal de fluorescence en fonction de la quantité d'antigène obtenu lors d'expérimentations,
- la figure 9 représente les lignes de courant d'un fluide contenant des particules magnétiques placé au contact d'un support comprenant deux domaines magnétiques aimantés dans des directions opposées,
- la figure 10A représente la distribution de complexes fluorescents capturés par des particules magnétiques au niveau de micro-sources de champ magnétique, déterminée lors d'expérimentations et observée par microscopie ; la figure 10B illustre le
rapport entre le signal spécifique et le signal non spécifique mesuré sur des images de microscopie du type de la figure 10A.

### DESCRIPTION DETAILLEE DE MODES DE REALISATION DE L'INVENTION

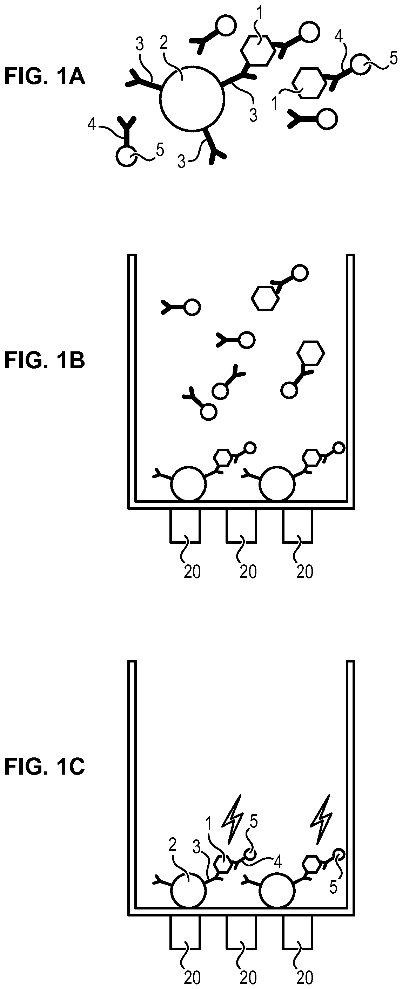

Les figures 1A à 1C illustrent de manière schématique les principales étapes du procédé de détection correspondant à un test d'immunodosage de type « sandwich ».

Dans ce cas, la molécule à détecter et à doser est un antigène.

Selon ce procédé, on réalise un mélange des éléments suivants :
- un échantillon susceptible de contenir l'antigène 1 dont on souhaite détecter et quantifier la présence,
- des particules magnétiques 2 préalablement couplées à un anticorps de capture 3, c'est-à-dire une protéine réceptrice spécifique de l'antigène à détecter, en excès par rapport à l'antigène,
- un anticorps de détection 4 portant un marqueur 5 fluorescent, luminescent ou coloré, en excès par rapport à l'antigène.

Ledit marqueur 5 peut être porté directement par l'anticorps de détection ou indirectement, par l'intermédiaire d'une enzyme.

Ledit mélange est réalisé dans un récipient, dans lequel, sous l'effet de la diffusion des différents constituants, l'antigène 1 se lie à l'anticorps de capture 3 couplé à une particule magnétique 2, puis l'anticorps de détection 4 se lie à l'antigène 1.

On forme ainsi un complexe « sandwich » formé d'une particule magnétique 2, de l'anticorps de capture 3, de l'antigène 1, de l'anticorps de détection 4 et du marqueur 5.

Les liaisons entre l'antigène et l'anticorps de capture, et entre l'anticorps de détection et l'antigène, sont des liaisons non covalentes.

En revanche, le couplage entre la particule magnétique et l'anticorps de capture est une liaison covalente.

Ledit mélange est déposé, par exemple au moyen d'une pipette, sur un support comprenant des micro-sources de champ magnétique ordonnées.

Les micro-sources de champ magnétique ont pour effet d'attirer les particules magnétiques et par conséquent d'immobiliser les complexes précités sur le support, selon un motif qui est étroitement lié à l'ordonnancement des micro-sources de champ magnétique par rapport à la surface dudit support. Par exemple, si les micro-sources de champ magnétique sont agencées sous forme d'un damier dont deux cases adjacentes présentent des aimantations de sens opposés, les particules magnétiques s'immobiliseront au niveau de la jonction entre deux micro-sources de champ magnétique, qui est l'endroit où le champ de fuite, ainsi que son gradient, est le plus intense.

Les antigènes non liés aux particules magnétiques et les anticorps marqués non liés à l'antigène ne sont pas attirés par les micro-sources de champ magnétique restent en suspension dans le mélange.

Une étape optionnelle de lavage, selon la description mais ne faisant pas partie de l'invention revendiquée, permet d'éliminer ces résidus et de ne conserver sur le support que les complexes, ceux-ci étant retenus par les micro-sources de champ magnétique par interaction avec les particules magnétiques.

En effet la force magnétique générée sur les complexes par les micro-sources de champ magnétique (qui sont capables de générer de très forts gradients de champ magnétique) est supérieure à la force d'aspiration exercée lors du lavage.

On a ainsi capturé et maintenu sur le support les molécules d'intérêt, couplées aux particules magnétiques.

Cependant, comme cela est exposé en détail plus bas, il est également possible de se dispenser de l'étape de lavage. En effet, la connaissance de l'ordonnancement des micro-sources de champ magnétique par rapport à la surface du support et la mise en oeuvre d'une technique de détection appropriée permettent de détecter les particules piégées par lesdites micro-sources ordonnées sans qu'il soit nécessaire de retirer l'échantillon ou de laver la surface du support.

Une fois cette capture réalisée, on peut alors mettre en oeuvre une méthode de quantification connue de l'homme du métier, selon la nature des marqueurs employés.

Parmi ces méthodes de quantification, on peut noter la fluorescence, la luminescence, la colorimétrie, une réaction RedOX, la radioactivité, la turbidimétrie, la détection magnétique par micro et nano-capteurs magnétiques GMR localisés, etc. Selon l'invention, la quantification est faite par fluorescence.

La durée totale de ce test est typiquement comprise entre 10 et 20 minutes, alors qu'il est compris entre 2 et 6 heures pour un test ELISA mettant en oeuvre les étapes classiques de mélange et de détection.

Par rapport au test ELISA qui, comme indiqué plus haut, est un procédé d'immunodosage en phase hétérogène, le procédé selon l'invention peut être considéré comme un procédé d'immunodosage en phase homogène.

En effet, la liaison entre l'antigène à doser et l'anticorps de détection se produit dans le volume de l'échantillon et non sur la surface du support, ledit support étant destiné uniquement à immobiliser les complexes pendant le lavage préalable à la détection et la quantification ou en vue d'une détection directe sans lavage telle qu'envisagée plus haut.

La présence, dans ledit volume, d'un grand nombre de particules magnétiques de dimensions qui peuvent être inférieures à ce qui est utilisé dans les procédés connus, et le coefficient de diffusion élevé que leur faible dimension confère à ces particules, augmente leur probabilité de rencontre avec les antigènes et les anticorps de détection.

On précise que, si l'exemple donné ci-dessus concerne un test d'immunodosage de type « sandwich », qui peut remplacer le test ELISA sandwich classique, le procédé peut être adapté pour offrir les mêmes modalités que d'autres tests d'immunodosage (du type ELISA direct ou ELISA par compétition) ou d'autres procédés impliquant la capture d'une molécule, tels que l'immunoprécipitation.

Par exemple, un test d'immunoprécipitation est destiné à purifier une protéine déterminée dans un échantillon comprenant une pluralité de protéines.

Ce test met en oeuvre des anticorps adaptés pour se lier sélectivement à la protéine que l'on souhaite détecter.

Dans un tel test, on couple lesdits anticorps à une pluralité de particules magnétiques et l'on introduit lesdites particules dans l'échantillon.

Les anticorps liés aux particules magnétiques vont alors se lier aux protéines qui sont reconnues spécifiquement.

Les protéines que l'on cherche à isoler sont liées aux anticorps qui sont eux-mêmes couplés aux particules magnétiques, formant ainsi des complexes.

Grâce aux micro-sources de champ magnétique ordonnées, il est alors possible d'immobiliser lesdits complexes sur un support puis, le cas échéant, de procéder à un lavage pour retirer le reste de l'échantillon.

Au contraire, dans les procédés ne mettant pas en oeuvre de sources de champ magnétique, les anticorps sont liés chimiquement au puits de la plaque de titration qui a été préalablement fonctionnalisé.

Par rapport au procédé de D. Issadore et al, l'utilisation d'un support comprenant des micro-sources de champ magnétique ordonnées permet, pour une taille donnée de particules magnétiques, d'augmenter la portée des micro-sources de champ magnétique et/ou, à portée donnée, d'augmenter la force exercée sur les particules de faibles dimensions.

Par conséquent, l'invention permet une capture plus rapide et plus efficace.

D'autre part, l'ordonnancement des micro-sources de champ magnétique permet de réaliser la détection sans nécessairement procéder au lavage du support, ce qui n'est pas possible lorsque les micro-sources de champ magnétique sont agencées aléatoirement dans le support, l'homme du métier n'étant alors pas en mesure de différencier les particules capturées des particules non capturées.

### Particules magnétiques

Les particules employées sont des particules magnétiques de dimensions micrométriques (microparticules) ou nanométriques (nanoparticules). Selon l'invention, les particules magnétiques ont un diamètre compris entre 5 nm et 500 nm.

De manière avantageuse, lesdites particules sont assimilables à des billes.

Dans la suite du texte, les particules pourront donc être désignées par le terme « billes » même si leur géométrie n'est pas une sphère parfaite.

De telles billes sont disponibles commercialement en grandes quantités et à faible coût, notamment pour des applications telles que l'imagerie par résonance magnétique (IRM) ce qui ne grève pas le coût du test.

De préférence, lesdites billes sont monodispersées, l'uniformité dimensionnelle des billes leur conférant des propriétés identiques et améliorant ainsi la diffusion des billes dans le mélange et leur capture par les micro-sources de champ magnétique.

Dans certains cas, les billes sont commercialisées sous une forme dispersée dans une matrice peu ou pas magnétique, telle qu'un polymère, de la silice (SiO₂), etc.

Les billes sont de préférence biocompatibles.

Pour permettre le couplage par liaison covalente d'un élément destiné à se coupler sélectivement avec la molécule à capturer, la surface des particules est fonctionnalisée, par exemple par des protéines A ou G.

Dans le présent texte, le terme « nanoparticule » désigne un objet dont les trois dimensions sont à l'échelle nanométrique, c'est-à-dire dont la taille caractéristique, par exemple le diamètre moyen s'il s'agit d'une bille, est typiquement inférieure à 100 nm.

Le terme « microparticule » désigne un objet dont les trois dimensions sont à l'échelle micrométrique, c'est-à-dire dont la taille caractéristique, par exemple le diamètre moyen s'il s'agit d'une bille, est compris entre 100 nm et 100 µm.

Sauf mention contraire, le terme « particule » ou « bille » employé dans la suite du texte englobe à la fois les nanoparticules et les microparticules magnétiques.

De par leurs dimensions, les particules magnétiques employées présentent des propriétés superparamagnétiques.

On rappelle que le terme « superparamagnétique » désigne la propriété de particules de matériau ferromagnétique ou ferrimagnétique de petites dimensions de changer aléatoirement de direction d'aimantation en l'absence d'un champ magnétique appliqué, sous l'effet de l'agitation thermique.

Le caractère d'être superparamagnétique implique qu'en l'absence de champ magnétique d'excitation extérieur, les billes n'ont aucun moment magnétique net, de sorte qu'elles ne s'attirent pas mutuellement, ce qui évite leur agglomération.

Par rapport aux microparticules, les nanoparticules, une fois couplées à un anticorps, présentent des performances beaucoup plus importantes en termes de capture des antigènes.

Cette amélioration peut s'expliquer par la réduction d'échelle des nanoparticules par rapport aux microparticules.

Ainsi, pour une réduction de diamètre d'un facteur 100 (par exemple entre une microbille de 3 µm de diamètre et une nanobille de 30 nm de diamètre) :
- le coefficient de diffusion est multiplié par 100,
- le rapport surface / volume est multiplié par 100,
- le nombre de billes par m³ est multiplié par 10⁶.

Les anticorps de capture se fixant sur la surface des particules, l'augmentation du rapport surface / volume permet, à masse de particules égale, d'augmenter le nombre d'anticorps de capture et par conséquent l'améliorer la sensibilité du test.

En outre, la diminution de la taille des particules accroît leur vitesse de diffusion dans le mélange et permet donc une formation plus rapide des complexes.

Un autre avantage des nanoparticules est qu'elles sont de dimensions équivalentes aux objets biologiques (brins d'ADN, marqueurs du cancer, protéines spécifiques) que l'on cherche à détecter.

Ainsi, elles sont animées du même mouvement brownien que ces objets, ce qui permet d'augmenter la capacité de les capturer.

Enfin, l'augmentation du nombre d'anticorps de capture grâce aux nanoparticules permet de déplacer l'équilibre thermodynamique de la réaction d'association et pourrait donc permettre l'utilisation d'anticorps actuellement négligés du fait de leur moindre affinité.

Par conséquent, bien que le procédé selon la description, mais ne faisant pas partie de l'invention, puisse être mis en oeuvre avec toute taille de particule magnétique, on choisira de préférence des particules ayant un diamètre compris entre 5 nm et 1 µm, de manière encore préférée entre 5 nm et 500 nm, voire entre 5 nm et 100 nm ou entre 5 et 50 nm.

### Micro-sources de champ magnétique

Par micro-source de champ magnétique, on entend un aimant dont les trois dimensions sont à l'échelle micrométrique, c'est-à-dire dont la longueur, la largeur et l'épaisseur sont inférieures à 1 mm.

Selon un mode de réalisation, lesdites micro-sources de champ magnétique sont constituées d'un matériau magnétique dur qui peut être aimanté de manière permanente.

Ceci permet d'assurer l'autonomie du dispositif de test, c'est-à-dire éviter tout besoin d'une alimentation électrique ou d'un champ magnétique externe pour permettre la capture des particules superparamagnétiques.

Cependant, il est envisageable que lesdites micro-sources soient réalisées en un matériau magnétique doux, une source de champ magnétique extérieur (par exemple un aimant permanent ou un électro-aimant) devant alors être placée au voisinage du support pour les aimanter, temporairement ou en permanence.

Le gradient de champ magnétique est en général le plus fort à la périphérie d'une micro-source de champ magnétique et, le cas échéant, à l'interface entre deux micro-sources de champ magnétique orientées différemment.

Les micro-sources de champ magnétique utilisées dans l'invention génèrent des champs magnétiques comparables à ceux des aimants macroscopiques actuellement disponibles, c'est-à-dire d'une intensité de l'ordre de 0,1 à 1×10⁶ A/m.

En revanche, les gradients de champ magnétique mesurés au-dessus de la surface du support peuvent atteindre 10⁶ T/m, c'est-à-dire plusieurs ordres de grandeur supérieurs aux gradients générés par des aimants macroscopiques.

Les forces magnétiques induites sur des particules magnétiques sont beaucoup plus importantes que celles générées par des aimants macroscopiques (c'est-à-dire présentant au moins deux dimensions supérieures à 1 mm), ce qui peut s'expliquer par le changement d'échelle.

Ainsi, la réduction d'un facteur 1000 de la source de champ magnétique (par exemple entre un aimant cubique de 10 mm de côté et un aimant de 10 µm) a pour effet de multiplier par 1000 la force magnétique exercée par la micro-source.

De ce fait, de telles micro-sources de champ magnétique sont à l'heure actuelle le seul moyen expérimentalement prouvé permettant de capturer efficacement des nanoparticules.

A ce jour, les inventeurs ont expérimentalement démontré que ces micro-sources de champ magnétique sont en effet capables de capturer des nanoparticules dont le diamètre est supérieur ou égal à 5 nm.

Cependant, l'intérêt des micro-sources de champ magnétique ne se limite pas aux nanoparticules superparamagnétiques.

En effet, même pour des microparticules, l'augmentation de la force magnétique par rapport à celle des aimants macroscopiques conventionnels facilite la capture des complexes comprenant les microparticules.

Les micro-sources de champ magnétique peuvent notamment être fabriquées par l'un des procédés suivants.

Selon un mode de réalisation de l'invention, les micro-sources sont fabriquées par structuration magnétique à l'échelle micrométrique d'une couche d'un matériau magnétique dur.

La réalisation d'aimants permanents performants aux échelles micrométriques peut comprendre les deux étapes successives suivantes :
- la synthèse de films magnétiques épais performants, présentant une épaisseur comprise entre 1 et 100 µm ;
- la réalisation de micro-sources de champ magnétique (assimilables à des micro-aimants permanents) par structuration magnétique dudit film magnétique.

### Synthèse de films magnétiques

Un procédé de dépôt par voie physique par pulvérisation triode qui permet le dépôt d'un matériau magnétique en couches épaisses (typiquement 1-100 µm) est décrit dans N.M. Dempsey et al., App. Phys. Lett. 90, 092509 (2007).

A titre d'exemple, des couches de NdFeB, de FePt et de SmCo ont été synthétisées et possèdent de remarquables propriétés magnétiques.

D'autres méthodes de fabrication d'aimants en couches peuvent être envisagés, en particulier les dépôts électrolytiques, les dépôts sol-gel, les dépôts par évaporation, le dépôt laser pulsé, etc.

On peut également procéder au collage d'une couche de matériau magnétique sur un substrat.

Les matériaux susceptibles d'être employés pour former ces couches sont des matériaux magnétiques durs, qui peuvent ensuite être aimantés de manière permanente.

Parmi les matériaux adaptés à cet usage, on peut citer les aimants à base de terres rares, de formule RFeB (où R consiste essentiellement en Nd, Pr, Tb, Dy ou un mélange de plusieurs de ces éléments), RCo ou RCoCu (structure cristallographique de type 1/5), RCoCuFe (structures cristallographiques de type 1/7 ou 2/17), RFeN (où R consiste essentiellement en Sm), les alliages métal de transition de la série 3d (Fe, Co, Ni)-métal noble (Pt ou Pd comme élément majoritaire), les aimants ferrite, les aimants MnBi, MnAl, MnGa, FeGa, AINiCo.

Selon un mode de réalisation avantageux, on choisit le matériau magnétique dur parmi les familles du NdFeB, du SmCo et du FePt.

Par ailleurs, la génération de champs magnétiques et de gradients de champs magnétiques importants requiert une microstructuration magnétique de la couche de matériau magnétique dur. C'est cette structuration qui assure l'ordonnancement des micro-sources de champ magnétique.

De manière particulièrement avantageuse, ladite couche peut être micro-structurée magnétiquement par l'une des deux techniques présentées ci-dessous.

### Structuration magnétique par méthode dite « topographique »

Cette technique consiste à structurer avant dépôt le substrat sur lequel le matériau magnétique dur est ensuite déposé et/ou à structurer directement la couche magnétique.

Les dimensions des structurations topographiques déterminent les dimensions des micro-aimants obtenus.

Chaque portion de couche magnétique gravée et/ou déposée sur un micro-plot ou dans une micro-cavité du substrat peut être assimilée à un élément magnétique indépendant.

Notons que des étapes de planarisation, de lithographie optique, d'attaque chimique peuvent être nécessaires.

Ces micro-éléments ainsi réalisés sont ensuite aimantés selon une direction choisie.

Ils constituent alors un ensemble de micro-aimants indépendants, présentant tous la même direction d'aimantation [A. Walther et al., J. Magn. Mag. Mat. 321 (2009) 590].

Ces micro-aimants individuels, éventuellement situés à différentes hauteurs, constituent des systèmes présentant de très fort gradients de champ magnétique aux échelles micrométriques [M. Kustov et al., J. App. Phys. 108, 063914 (2010)].

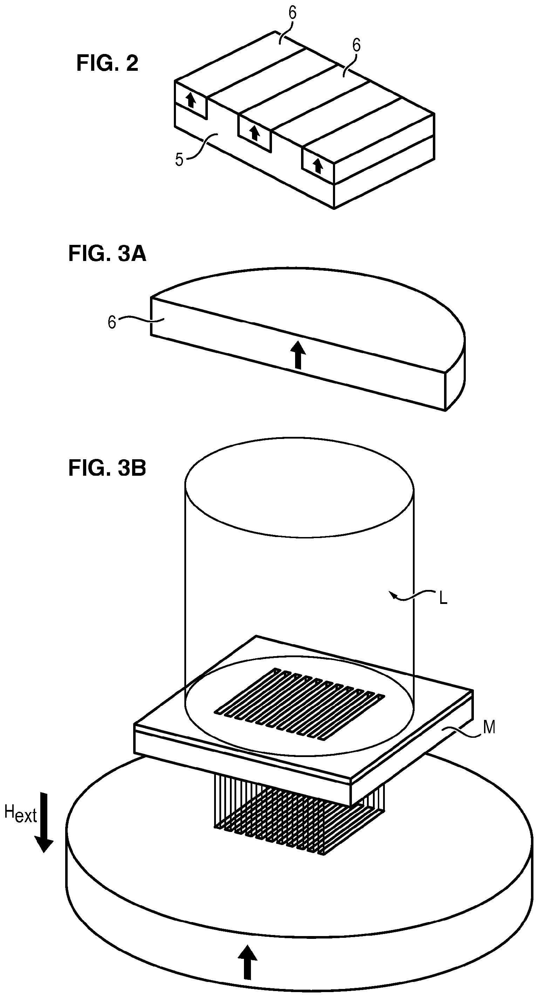

La figure 2 illustre de manière schématique un exemple de microstructuration topographique.

Le substrat 5 comprend une pluralité de micro-cavités qui, dans cet exemple, se présentent sous la forme de tranchées parallèles.

Le matériau magnétique dur est ensuite déposé dans ces tranchées de sorte à former des régions magnétiques parallélépipédiques 6.

La formation de telles cavités et le dépôt de matériau magnétique sont en eux-mêmes connus de l'homme du métier et ne seront donc pas décrits en détail ici.

Le cas échéant, on procède ensuite à un polissage de sorte à obtenir une plaque plane dont la surface présente une alternance de régions du substrat 5 et des régions de matériau magnétique dur 6.

On applique ensuite un champ magnétique externe de sorte à aimanter de manière permanente les régions 6 de matériau magnétique dur.

L'homme du métier est à même de définir l'intensité du champ magnétique à appliquer pour obtenir l'aimantation souhaitée en fonction des matériaux employés.

De telles micro-sources de champ magnétique génèrent un fort gradient de champ magnétique et sont donc adaptées à la capture et à l'immobilisation des nano ou microparticules magnétiques.

Les dimensions des micro-sources sont à l'échelle sub-millimétrique et dépendent de la précision des procédés topographiques employés.

Il va de soi que les formes de ces micro-sources ne sont pas limitées à des formes parallélépipédiques ; elles peuvent ainsi être agencées sous la forme d'un échiquier, mais aussi présenter une forme circulaire, hexagonale, etc.

### Structuration magnétique par méthode dite « empreinte thermo-magnétique » (ou « Thermo-MagneticPatterning » selon la terminologie anglo-saxonne)

Le principe de la structuration par empreinte thermo-magnétique est d'utiliser une source de chaleur pour chauffer localement certaines zones d'une couche magnétiquement dure et ainsi créer des volumes aimantés selon des directions alternées, constituant des micro-sources de champ magnétique.

A titre d'exemple, on peut utiliser comme source de chaleur un laser impulsionnel nanoseconde selon une méthode dont le principe est décrit en référence aux figures 3A à 3D.

Comme illustré à la figure 3A, une couche 6 d'un matériau magnétique dur est aimantée dans une direction et un sens donné (schématisé par la flèche).

Cette couche 6 est ensuite placée dans un champ magnétique externe uniforme *Hₑₓₜ* (*µ₀Hₑₓₜ* < *µ₀H_{c}*) de direction opposée à la direction d'aimantation originelle, puis elle est localement irradiée par un laser L impulsionnel (dans notre cas un laser à excimères de type KrF (248 nm), ou un laser Nd-YAG).

Comme illustré à la figure 3B, ladite irradiation est réalisée à travers un masque M qui, dans cet exemple, est pourvu d'un certain nombre d'ouvertures rectangulaires parallèles.

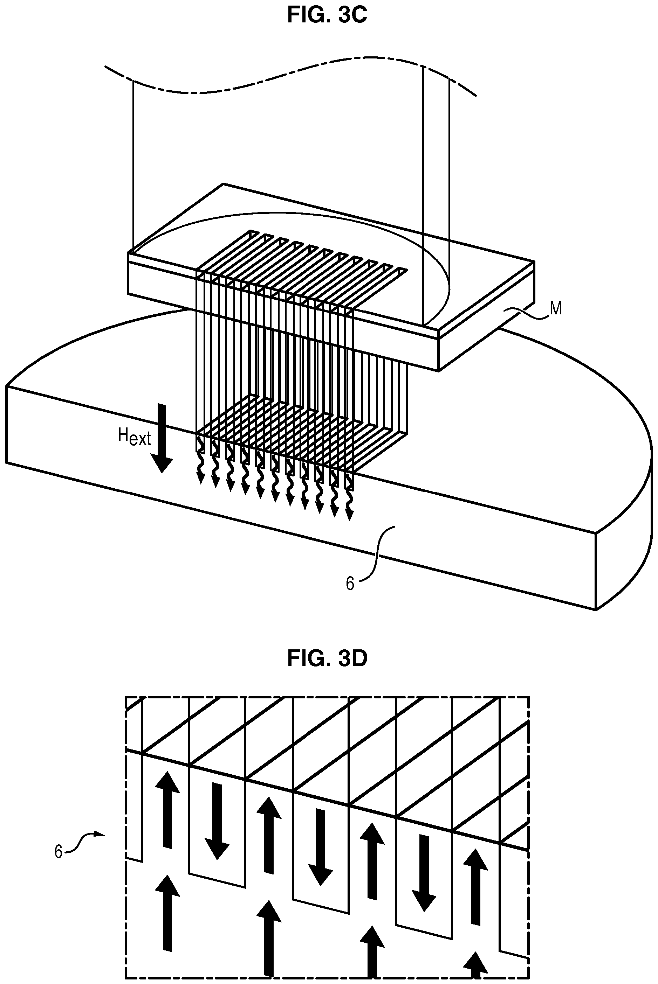

La température à la surface des zones irradiées augmente très rapidement puis la chaleur se diffuse dans le matériau (cf. figure 3C).

Etant donné la diminution du champ coercitif *µ₀H_{c}* d'un matériau lorsque sa température augmente, le renversement magnétique des zones irradiées peut être obtenu par application durant l'impulsion laser du champ magnétique externe*Hₑₓₜ*.

Comme illustrée à la figure 3D, la couche est constituée in fine d'un réseau de micro-aimants d'aimantations alternées schématisées par les flèches et de dimensions définies par les dimensions du masque utilisé durant l'irradiation laser.

Les systèmes réalisés par ce procédé présentent de très forts gradients de champ magnétique aux échelles micrométriques [M. Kustov et al., J. App. Phys. 108, 063914 (2010)].

Pour davantage de détails concernant ce procédé, on pourra se référer à l'article de F. Dumas-Bouchiat et al., App. Phys. Lett. 96, 102511 (2010).

Ce principe d'empreinte thermo-magnétique peut être étendu à toutes sortes de couches magnétiques dures y compris celles présentant des aimantations isotropes ou dans le plan.

### Procédé dit « Micro Magnetic Imprinting »

Selon une autre forme d'exécution de l'invention, les micro-sources 21 se présentent sous la forme d'agglomérats ordonnés de nano ou microparticules d'un matériau magnétique dur enfermées dans une matrice non magnétique et aimantées.

De manière particulièrement avantageuse, ladite matrice peut être en un matériau flexible, ce qui permet d'obtenir un support souple.

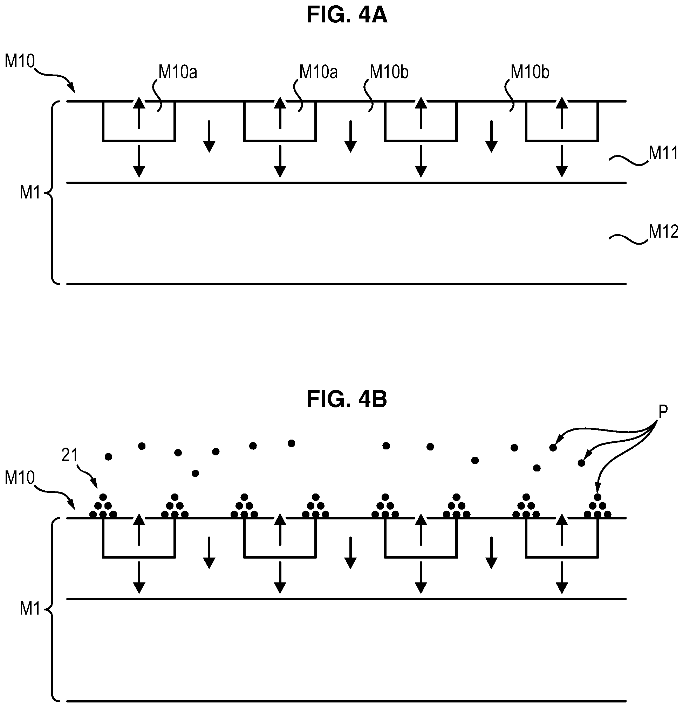

Les figures 4A à 4D illustrent des étapes d'un mode de fabrication du support selon l'invention.

En référence à la figure 4A, on utilise un substrat maître M1 pour ordonner des nano ou microparticules avant de les enfermer dans une matrice non magnétique.

Ledit substrat maître M1 présente une face magnétiquement structurée, c'est-à-dire une face M10 constituée d'une pluralité de micro-sources M10a, M10b de champ magnétique réparties selon un motif déterminé.

L'aimantation des différentes micro-sources est schématisée par une flèche.

Sur les figures 4A à 4D, la face magnétiquement structurée M10 du substrat maître est plane.

Cependant, dans certaines formes d'exécution de l'invention, il peut être préférable d'employer un substrat maître dont la face n'est pas plane mais présente des cavités ou des plots, afin de reproduire dans le film formé à partir du substrat maître des plots ou des cavités complémentaires.

Le substrat maître peut être fabriqué par différentes techniques connues de l'homme du métier.

En particulier, on peut mettre en oeuvre un procédé de structuration par empreinte thermo-magnétique ou un procédé de structuration par topographie tels que décrits plus haut.

Dans un exemple non limitatif illustré à la figure 4A, le substrat maître est constitué d'une couche M11 magnétiquement dure de NdFeB présentant une épaisseur de 5 µm sur un substrat support M12 de silicium et magnétiquement structurée par empreinte thermo-magnétique de sorte à former des régions M10a d'aimantation opposée à celle des régions M10b.

Dans ce substrat maître, l'intensité du champ magnétique et du gradient de champ magnétique est maximale au niveau des interfaces entre les régions M10a, M10b présentant des aimantations opposées.

Par conséquent, des nano ou microparticules présentant une susceptibilité magnétique positive sont attirées par la force magnétophorétique vers ces interfaces.

Le substrat maître n'est cependant pas limité à cette forme particulière mais peut être constitué d'un matériau magnétique dur ou doux structuré par topographie.

Le substrat maître peut éventuellement être un conducteur électrique.

Lorsque le substrat maître présente une surface structurée par topographie, c'est-à-dire non plane, cette topographie particulière peut être mise à profit pour imprimer une forme complémentaire au film final.

Lorsque l'on souhaite former un film plan à partir d'un tel substrat maître, il est nécessaire de planariser celui-ci au préalable, soit par retrait de matière (par exemple au moyen d'un polissage mécano-chimique pour supprimer les plots), soit par ajout de matière (par exemple au moyen d'une technique de dépôt pour combler les cavités).

En référence à la figure 4B, on envoie sur la face magnétiquement structurée du substrat maître 1 des nano ou des microparticules P d'un matériau magnétique dur.

Sous l'effet de la force magnétophorétique attractive de la face magnétiquement structurée du substrat maître, les particules P s'agglomèrent aux bords de chacune des micro-sources.

Dans la mesure où la force décroît au fur et à mesure que l'on s'éloigne de la face du substrat maître, ces agglomérats 21 présentent généralement une section triangulaire ou trapézoïdale, avec une base plus large du côté du substrat maître M1 et diminuant avec la distance.

Pour favoriser l'ordonnancement des particules non uniquement dans un plan mais aussi dans une direction perpendiculaire au substrat maître, il est possible d'appliquer simultanément un champ magnétique externe présentant une direction appropriée.

Lors de l'envoi des particules, on agite avantageusement le substrat maître de manière à optimiser le piégeage des particules au niveau des bords des micro-sources de champ magnétique.

Par ailleurs, pendant ou après cet envoi, on peut projeter un jet de gaz sec pour favoriser le piégeage des particules au niveau desdits bords et/ou éliminer les particules non piégées avant le dépôt de la matrice non magnétique.

Les particules sont en un matériau magnétique dur, qui peut être l'un des matériaux de la liste développée plus haut.

Par conséquent, l'application, après la formation du film, d'un champ magnétique externe permettra de les aimanter de manière permanente et ainsi de former un film magnétiquement structuré autonome.

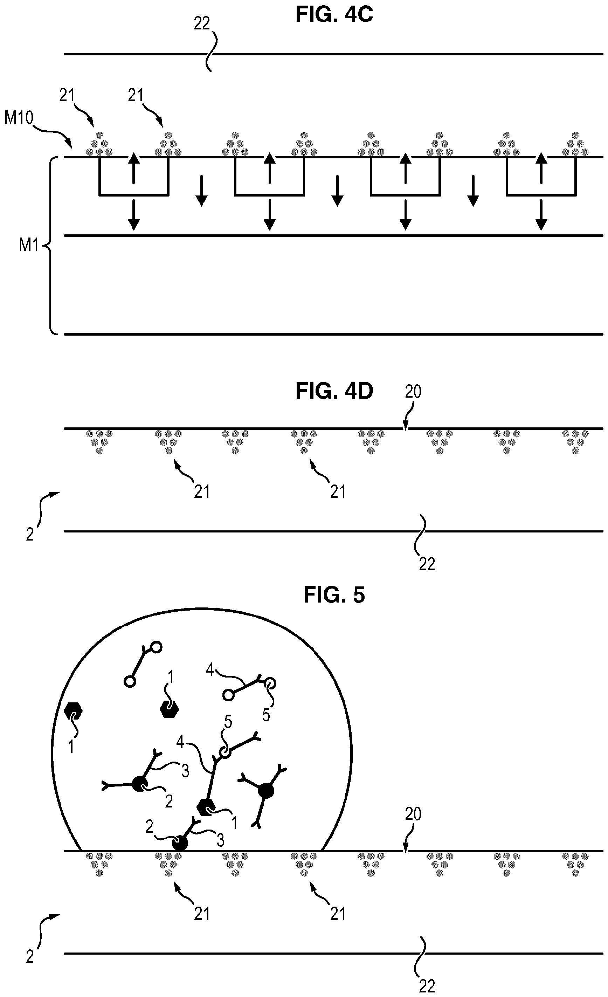

En référence à la figure 4C, on dépose ensuite, sur les particules agglomérées sur le substrat maître, une matrice 22 en un matériau non magnétique sous la forme d'une couche mince.

L'épaisseur de la matrice 22 est typiquement comprise entre 100 nm et 5 mm

De manière avantageuse, la matrice est en un matériau élastomère, ce qui permet de conférer au film une certaine flexibilité.

Selon des modes de réalisation préférés de l'invention, la matrice est en l'un des matériaux suivants : du polydiméthylsiloxane (PDMS), de la résine époxy, du caoutchouc, de la bakélite, du polyméthacrylatede méthyle (PMMA), du polystyrène (PS), de la résine photosensible, du parylène, des oxydes tel que SiO₂, Al₂O₃, des métaux tels que Cu, Ag, des matériaux carbonés tels que le graphite, les DLC, etc.

Si nécessaire, on laisse la matrice 22 durcir ou réticuler pendant la durée appropriée.

Par ailleurs, la matrice peut être avantageusement être en un matériau transparent ou translucide.

L'homme du métier est à même de sélectionner une matrice appropriée parmi les produits disponibles sur le marché en fonction des propriétés recherchées.

En référence à la figure 4D, on délamine le film 2 constitué de la matrice 22 et des agglomérats 21 de particules magnétiques vis-à-vis du substrat maître M1.

Le substrat maître peut quant à lui être réutilisé pour la fabrication d'un nouveau film.

Ainsi, bien que le substrat maître nécessite la mise en oeuvre de techniques de microfabrication et présente par conséquent un certain coût, il peut être réutilisé indéfiniment et la fabrication du film magnétiquement structuré lui-même, qui n'implique

pas de telles techniques complexes et onéreuses, ne met en oeuvre que des matériaux peu coûteux.

Ce procédé est en outre aisément industrialisable et permet de réaliser des films de grande surface et en grande quantité à bas coût.

Selon une forme d'exécution de l'invention, on peut déposer sur le substrat maître, avant de déposer les particules, une couche d'un matériau permettant de faciliter le délaminage du film et qui reste solidaire du substrat maître lors du délaminage.

Compte tenu de la faible taille des particules à immobiliser sur le support, les microsources de champ magnétique ordonnées sont de préférence agencées directement à la surface du support - ou au voisinage immédiat de celle-ci - en contact avec l'échantillon pour procurer des forces d'attraction maximales.

### Plaque multi-puits

De manière conventionnelle, des plaques de titration comprenant une pluralité de puits (par exemple 96 ou 384 puits) sont utilisées dans les tests d'immunodétection.

Pour immobiliser les complexes, on peut intégrer à une telle plaque des microsources de champ magnétique ordonnées telles que décrites ci-dessus.

Cette intégration peut être obtenue en réalisant la plaque elle-même dans un matériau adapté pour former des micro-sources de champ magnétique ordonnées, selon l'un des procédés décrits plus haut. Les micro-sources de champ magnétique sont alors placées de préférence au fond de chacun des puits.

Selon une autre forme d'exécution, mais ne faisant pas partie de l'invention revendiquée, il est possible d'insérer à l'intérieur de chaque puits un support de la dimension dudit puits et sur lequel sont agencées lesdites microsources de champ magnétique ordonnées.

Selon une autre forme de réalisation, mais ne faisant pas partie de l'invention revendiquée, il est possible de réaliser une plaque de titration dans laquelle les puits n'ont pas de fond, et de rapporter sous ladite plaque un support plan contenant des micro-sources de champ magnétique ordonnées agencées au niveau de chaque puits, de sorte à constituer le fond étanche desdits puits.

Ainsi, le procédé est compatible avec les supports usuellement employés en immunodosage, ce qui ne nécessite pas de modification des appareils de laboratoire existants.

### Support plan

Selon un mode de réalisation particulièrement avantageux de la description, mais ne faisant pas partie de l'invention revendiquée, le support sur lequel on immobilise les complexes n'est pas une plaque de titration mais un support plan à la surface duquel sont agencées une pluralité de micro-sources magnétiques ordonnées.

Un tel support peut être réalisé par les divers procédés de micro-structuration de couches magnétiques décrits plus haut.

De manière alternative, ledit support peut être réalisé par le procédé « Micro Magnetic Imprinting » décrit plus haut.

Cette technique de fabrication présente l'avantage de pouvoir fabriquer à bas coût une grande quantité de supports, seul le substrat maître, qui est réutilisable à volonté, étant onéreux.

Par ailleurs, avec cette technique, le support présente l'avantage de pouvoir être réalisé en un matériau transparent ou translucide.

Cette propriété peut dans certains cas faciliter la détection et la quantification par méthodes optiques.

Ainsi, elle permet d'éclairer le support par-dessous, ce qui permet d'observer la coloration par le dessus du support avec une caméra et des filtres que l'homme du métier saura choisir.

Au contraire, cette transparence peut être gênante par exemple dans le cas de la fluorescence intense, où les signaux pourraient interférer entre emplacements contigus.

On pourra donc choisir le matériau du support en fonction de la méthode de détection envisagée.

Lorsqu'un tel support plan est utilisé, le mélange de la molécule à détecter, des particules magnétiques et de l'élément apte à se lier sélectivement à ladite molécule est réalisé dans un récipient, puis des volumes limités de mélange sont prélevés et déposés sur ledit support sous forme de goutte, comme illustré sur la figure 5.

La taille des gouttes dépend du volume déposé et de l'hydrophilie du matériau du support. Cette taille est donc ajustable, typiquement compris entre 1 microlitre et 200 microlitres.

Les micro-sources de champ magnétique ordonnées sont avantageusement réparties sur la surface du support selon un motif régulier, de sorte à permettre la capture des complexes quelle que soit la région du support sur laquelle la goutte a été déposée.

L'emplacement destiné à chaque goutte est de préférence matérialisé sur le support plan, par exemple par un marquage magnétique local, ou chimiquement (en rendant la surface du support localement hydrophile ou hydrophobe), ou encore topographiquement, par un creux de faible profondeur réalisé dans la surface du support. Il est également possible de marquer visuellement l'emplacement pour aider l'expérimentateur à le localiser.

De manière inattendue, il a été démontré que la vitesse de capture des billes magnétiques était sensiblement plus élevée dans des gouttes déposées sur un tel support plan que dans une solution déposée dans un puits d'une plaque de titration.

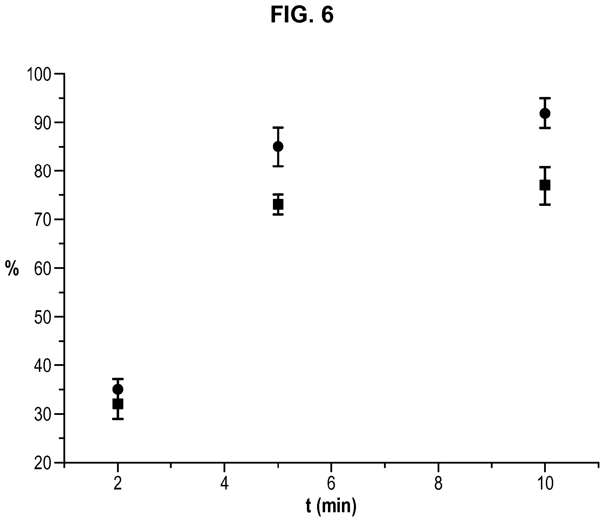

La figure 6 présente le pourcentage de billes de 100 nm de diamètre capturées respectivement dans une goutte (données représentées par un disque) et la quantité de billes du même type capturées dans un puits (données représentées par un carré).

Au bout de 10 minutes, 75% des billes ont été capturées par les micro-sources de champ magnétique ordonnées disposées au fond du puits de titration, tandis que plus de 90% des billes ont été récupérées par les micro-sources de champ magnétique ordonnées disposées dans le support recevant la goutte.

Ce phénomène est expliqué par la présence d'un mouvement de convection induit à l'intérieur du fluide par le convoyage des particules magnétiques vers les micro-sources de champ magnétique ordonnées qui les capturent.

La rapidité de diffusion des particules permet donc de s'affranchir de tout dispositif de vibration qui est nécessaire dans les tests conventionnels pour accélérer le mélange de la solution.

Un autre avantage du support plan par rapport à une plaque de titration conventionnelle est qu'il permet, pour un même encombrement du support, de fournir davantage de sites de capture des molécules.

### Cinétique de capture de nanoparticules magnétiques par un support comprenant des micro-sources de champ magnétique ordonnées

La cinétique et l'efficacité de capture remarquable des nanoparticules magnétiques par des supports comprenant des micro-sources de champ magnétique ordonnées sont dues à un couplage hydrodynamique entre les particules.

En effet, les particules magnétiques les plus proches de la surface du support sont attirées rapidement par les micro-sources de champ magnétique ordonnées, et leur traînée entraîne hydrodynamiquement le mouvement de particules plus éloignées vers les micro-sources de champ magnétique, qui, une fois rapprochées de la surface du support, se trouvent à leur tour attirées magnétiquement.

Ce couplage entre forces magnétiques et entraînement hydrodynamique a pour conséquence l'apparition d'un courant fluidique de convection qui brasse le fluide au-dessus des micro-sources de champ magnétique, ce qui explique l'efficacité et la cinétique de capture à longue distance des nanoparticules, malgré la faible taille de celles-ci.

Cette interprétation est justifiée par un modèle théorique et par une expérience démontrant l'existence de ce couplage.

Dans cette expérience, les inventeurs ont visualisé le mouvement d'un échantillon fluide grâce à des particules colorées non magnétiques en suspension dans un fluide déposé sur une jonction linéaire entre deux zones aimantées.

Cette jonction simule un élément d'une matrice de micro-sources de champ magnétique ordonnées réalisée par la technique Thermo Magnetic Patterning décrite plus haut.

C'est à cette jonction que sont localisés les gradients de champ magnétiques les plus élevés.

La mesure du mouvement et de la vitesse des billes (par une technique dite « particle tracking velocimetry ») a permis de reconstituer le mouvement et la vitesse du fluide.

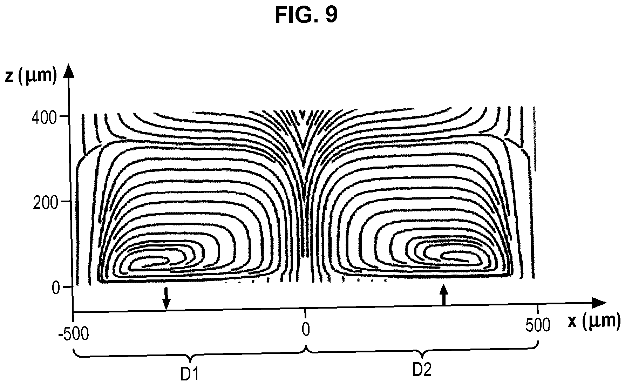

Sur la figure 9, qui illustre les lignes de courant induites par l'attraction de nanoparticules superparamagnétiques en suspension au-dessus d'une jonction entre deux domaines magnétiques D1 , D2 dont les sens d'aimantation sont représentés par des flèches (l'axe z désignant l'épaisseur de fluide au-dessus de la jonction), on voit clairement que deux vortex symétriques se créent rapidement de part et d'autre de la jonction, qui brassent le liquide sur une distance de l'ordre de plusieurs mm.

En absence de microparticules magnétiques, aucun mouvement ordonné du fluide n'est apparent.

Le champ des vitesses du fluide mesuré correspond, à 10% près, à ce qui est calculé par le modèle de couplage hydrodynamique - magnétique décrit ci-dessus.

Ce couplage se manifeste pour des concentrations de particules magnétiques supérieures à 10⁶ particules/ml, ce qui est effectivement observé expérimentalement.

La formation des courants de convection est intimement liée à l'ordonnancement des micro-sources de champ magnétique.

### Système microfluidique

Selon l'invention, le support est un système microfluidique de type « lab-on-chip ».

Le support comprend alors un microcanal et un réseau de micro-sources magnétiques agencées de manière ordonnée dans au moins une paroi dudit canal.

Ledit microcanal peut être réalisé selon le procédé « Micro Magnetic Imprinting » décrit plus haut.

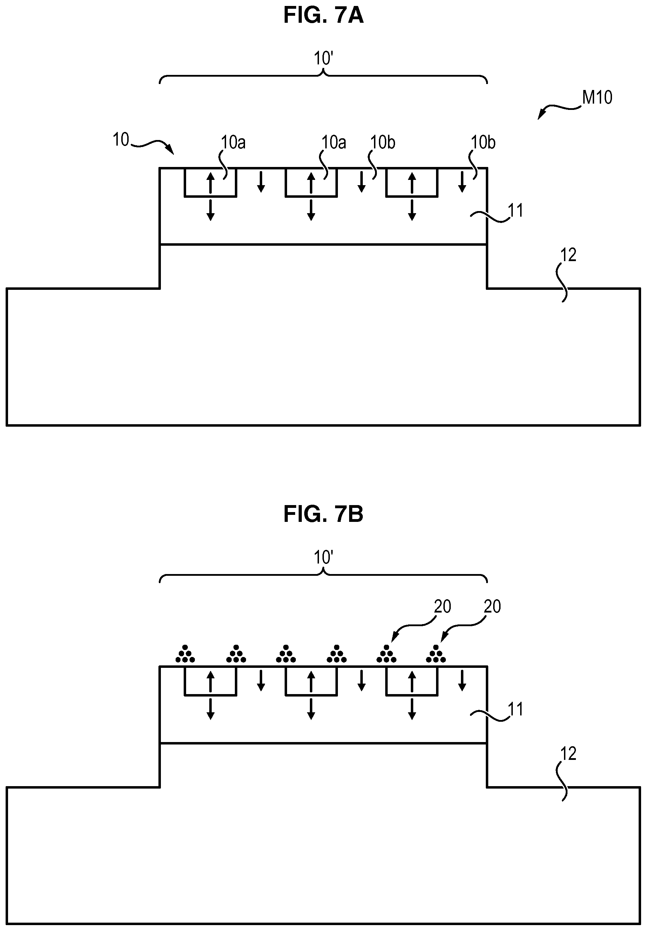

Dans ce cas, comme illustré aux figures 7A à 7E, la face magnétiquement structurée du substrat maître 1 n'est pas plane mais présente au moins un plot ou une cavité dont le négatif sera présent sur le film.

Dans l'exemple non limitatif illustré à la figure 7A, la face magnétiquement structurée 10 du substrat maître 1 présente un plot en relief 10', la surface du plot étant constituée d'une pluralité de micro-sources de champ magnétique 10a, 10b.

Par exemple, ledit plot peut présenter une forme parallélépipédique.

Selon les applications visées, le reste de la surface du substrat maître peut également être magnétiquement structurée, mais il est aussi envisageable que seule la surface du plot 10' soit magnétiquement structurée ; dans ce dernier cas, les nano ou microparticules apportées ne s'aggloméreront qu'aux bords des micro-sources magnétiques constituant la surface du plot 10', le reste de la surface du substrat maître ne retenant pas de particules.

Comme expliqué plus haut, on apporte sur le substrat maître les nano ou microparticules magnétiques, qui s'ordonnent et s'agglomèrent en structures tridimensionnelles 20 aux bords des micro-sources de champ magnétique 10a, 10b (figure 7B).

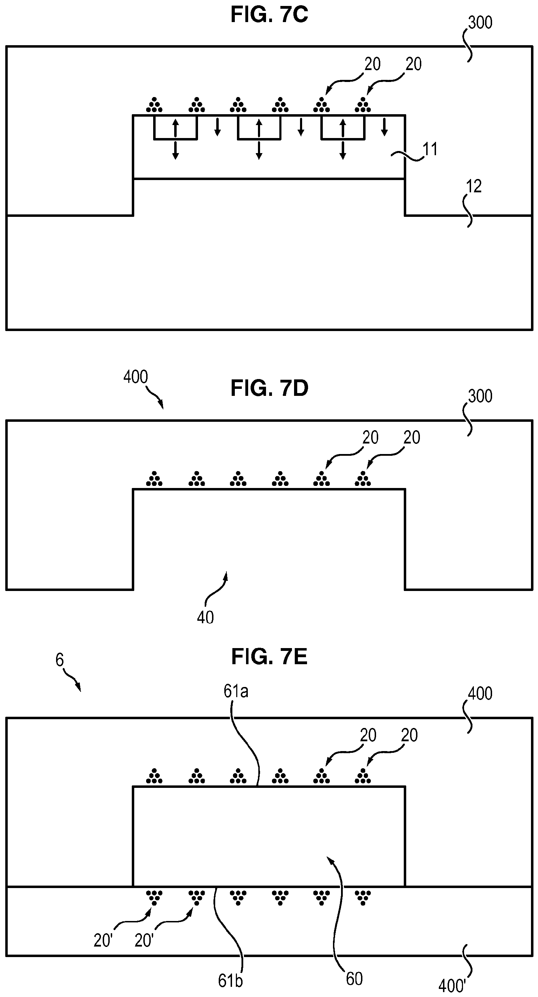

Ensuite, en référence à la figure 7C, on dépose la matrice non magnétique 3 sur le substrat maître.

A l'issue du délaminage, le film 4 ainsi obtenu illustré à la figure 7D présente donc une cavité 40 complémentaire du plot 10' du substrat maître.

Selon la forme du bossage, le film comporte ainsi un ou plusieurs puits, ou un ou plusieurs canaux.

A partir d'un tel film, on peut donc former un micro-canal fluidique.

A cet effet, comme illustré à la figure 7E, il suffit d'appliquer contre ledit film 4 un film plan 4' ou toute autre structure permettant de former la quatrième paroi de la cavité 40.

De manière avantageuse, ledit film plan 4' peut également être fabriqué selon l'invention et comprendre à sa surface des structures magnétiques tridimensionnelles 20'.

Ainsi, on forme à partir des films 4 et 4', un dispositif 6 comprenant un micro-canal 60 qui présente, sur deux parois opposées 61a et 61b, une surface magnétiquement structurée.

Un traitement de surface peut permettre d'assurer l'étanchéité des deux films.

Par exemple, lorsque les deux films possèdent une matrice en PDMS, on peut effectuer une activation des surfaces par un plasma d'oxygène.

Grâce à la présence de structures magnétiques tridimensionnelles sur les deux faces 61a et 61b du micro-canal 61, le piégeage de nano ou microparticules dans une solution circulant dans ledit micro-canal est améliorée.

Naturellement, la nature des particules magnétiques, ainsi que leur répartition par rapport à la surface de chaque paroi, peuvent être identiques pour chacune des deux parois ou bien être différentes.

Dans un tel système, on injecte le mélange à l'entrée du microcanal : la solution circule dans le microcanal, les complexes étant progressivement immobilisés sur le fond du microcanal par les micro-sources de champ magnétique.

On peut ensuite éventuellement procéder à une étape de lavage pour retirer les résidus de la solution, les complexes restant magnétiquement piégés par les micro-sources situées sur les parois du canal.

### Exemple de couplage d'un anticorps à une particule magnétique (protocole commercial Dynabead)

A titre indicatif, le couplage d'un anticorps à une bille magnétique peut être réalisé selon la procédure suivante :
- suspendre à nouveau les billes en agitant au vortex une solution dans laquelle elles sont dispersées,
- prélever la quantité de billes souhaitée,
- retirer le surnageant à l'aide d'une pipette et d'un aimant,
- ajouter l'anticorps dilué dans du PBS-Tween 0,01%,
- incuber 10 minutes à température ambiante avec une agitation,
- retirer le surnageant à l'aide d'une pipette et d'un aimant,
- resuspendre les billes couplées à l'anticorps dans du PBS-Tween 0,01%.

### Validations expérimentales

Un mode de réalisation de l'invention a été validé dans un test de dosage d'un antigène de type « sandwich » réalisé dans les conditions suivantes.

On a utilisé pour cette expérience comme antigène à doser la « coreprotein » (biotinylée) du virus de l'hépatite C, comme anticorps de capture un anticorps monoclonal spécifique de cette protéine (Pierce Hepatitis C Virus Core Antigen Monoclonal Antibody (C7-50)), des billes superparamagnétiques de 100 nm de diamètre, fonctionnalisées avec de la protéine A (fluidMAG-Protein A, Chemicell) et comme anticorps de détection des quantum dots fonctionnalisés avec de l'avidine (QD625, Invitrogen).

Dans cette expérience, les concentrations utilisées étaient de 1,3 nM pour l'anticorps de capture, 10⁹/ml pour les billes superparamagnétiques et 10¹⁰/ml pour les quantum dots.

Les quantum dots, les billes fonctionnalisées par l'anticorps et l'antigène biotinylé ont été placés, lors d'une même étape, sur un substrat souple comprenant des micro-sources de champ magnétique.

### La durée de mélange/capture a été fixée à 7 minutes.

Après ce temps, le surnageant a été retiré et deux lavages ont été effectués avec 50 µl de PBS.

Enfin, la quantité d'antigène a été mesurée directement sur le support grâce à la fluorescence des quantum dots.

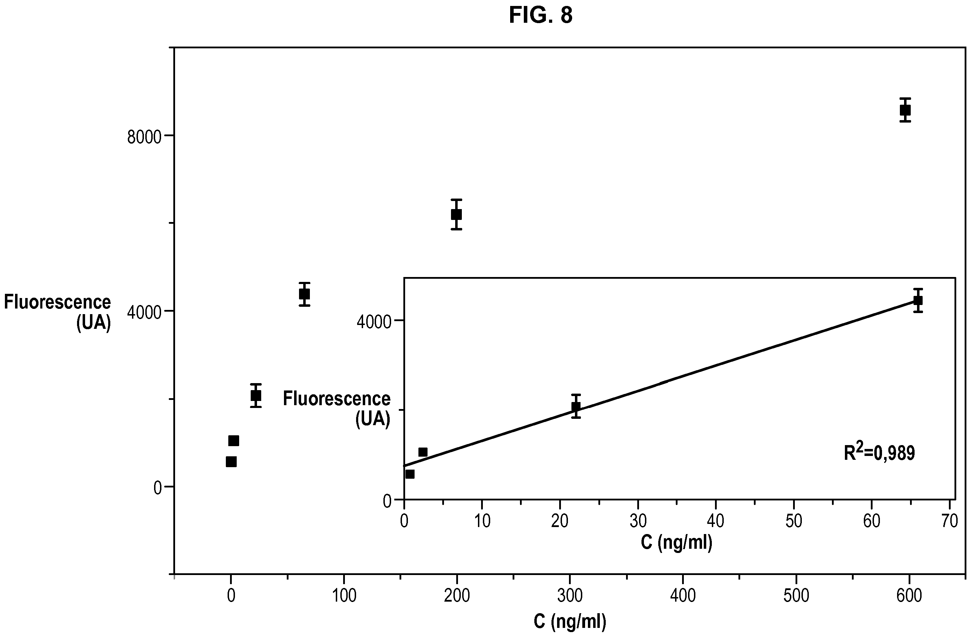

La figure 8 illustre la fluorescence (en unités arbitraires) en fonction de la concentration en antigène.

### L'ensemble du test a été réalisé en une dizaine de minutes.

La limite de détection est de 10 pM, ce qui est comparable à la limite des tests ELISA disponibles sur le marché menés sur cette protéine.

Au-delà de 5 nM, le teste était positif, mais la réponse du test était saturée à cause la caméra utilisée.

En tout état de cause, de telles concentrations sont bien au-delà des cas cliniques ; en outre il est possible de diluer l'échantillon pour refaire un test si nécessaire.

### Détection sans lavage

L'organisation spatiale des micro-sources de champ magnétique ordonnées permet de faire une détection sans lavage du support ni marquage des immuno-complexes.

Il suffit pour cela de déterminer la densité de fluorescence à la surface des micro-sources de champ magnétique ordonnées dans les zones de gradient de champ magnétique fort (signal spécifique) et dans les zones de gradient de champ magnétique faible (signal non-spécifique).

Selon la description, mais ne faisant pas partie de l'invention revendiquée, cette détection peut se faire par fluorescence, en focalisant la prise d'image sur la surface du support contenant les micro-sources de champ magnétique, ou en disposant des détecteurs à surface du support lui-même.

Selon l'invention, cette détection est réalisée par fluorescence, en focalisant la prise d'image sur la surface du support contenant les micro-sources de champ magnétique, ou en disposant des détecteurs à surface du support lui-même.

L'expérience suivante démontre la faisabilité d'une telle détection.

Des nanoparticules magnétiques (50 nm, 10⁹ ml⁻¹) et des nanoparticules fluorescentes non magnétiques (quantum dots, 30 nm, 10⁹ ml⁻¹) fonctionnalisées par de la biotine ont été mélangées à des concentrations faibles d'avidine, qui est une protéine qui se lie de façon quasi-irréversible à quatre molécules de biotine.

Ce couplage mime la formation d'immuno-complexes (nanoparticules magnétique)-(anticorps de capture)-(antigène)-(anticorps de détection)-(marqueur fluorescent) ou autre.

Après 10 minutes d'incubation, une goutte est déposée sur la surface d'un support comprenant des micro-sources de champ magnétique ordonnées réalisé par la technique de Micro-Magnetic Imprinting et observée au microscope optique de fluorescence 10 minutes après.

Le microscope est focalisé au niveau du support.

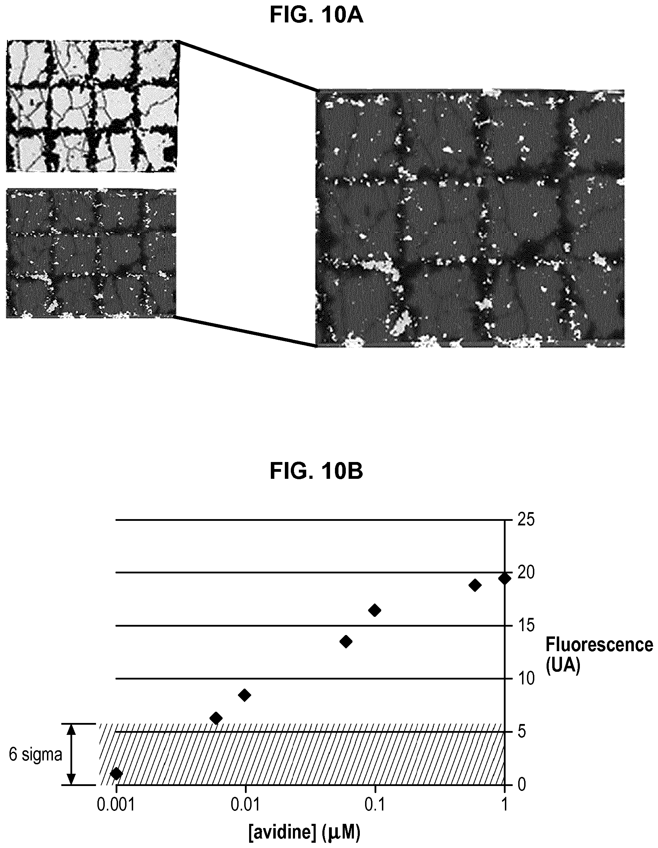

Sur la figure 10A, l'image en haut à gauche présente la structure du support comprenant les micro-sources de champ magnétique ordonnées observée en contraste de phase (les micro-sources de champ magnétique étant agencées selon un quadrillage qui présente une couleur plus foncée que le reste de la matrice) tandis que l'image en bas à gauche présente la distribution des quantum dots biotinylés à la surface du support en présence de nanoparticules magnétiques biotinylées et d'avidine, observée en fluorescence (les quantum dots présentent une couleur claire sur cette image).

La superposition des deux images à droite de la figure 10A permet de voir les quantum dots issus d'une interaction spécifique avec les nanoparticules magnétiques : on voit clairement que les quantum dots (de couleur claire) sont principalement alignés le long du quadrillage (de couleur sombre) défini par les micro-sources de champ magnétique (signal spécifique) et reproduisent donc le motif défini par les micro-sources de champ magnétique, seuls quelques quantum dots se trouvant sur des zones situées à l'intérieur de ce quadrillage (signal non spécifique).

En absence d'avidine, aucun motif n'est visible.

La densité de fluorescence sur les zones de fort et faible gradient de champ magnétique a été mesurée sur les images de microscopie montrées en A.

La figure 10B montre l'évolution du rapport (signal spécifique) / (signal non spécifique), qui est égal au rapport (fluorescence spécifique x aire des régions à faible gradient de champ magnétique) / (fluorescence non spécifique x aire des régions à fort gradient de champ magnétique) en fonction de la concentration d'avidine.

Si aucune interaction spécifique n'a lieu, ce rapport est de 1. La zone hachurée correspond à l'intervalle de confiance à 6 sigmas. Tous les points en dehors de cette zone sont significatifs avec une probabilité d'erreur inférieure à 10⁻⁶.

Une concentration de 50 nM est donc détectable au seuil de signification 99,9997% (six sigmas).

La microstructuration des micro-sources de champ magnétique ordonnées permet de définir les régions d'intérêt « spécifiques » et « non-spécifiques ». Elle peut donc être mise à profit pour simplifier la détection.

Il faut noter que des micro-sources de champ magnétique qui seraient disposées de manière aléatoire ne permettraient pas cette détection sans lavage.

### REFERENCES

D. Issadore et al, Self-assembled magnetic filter for highly efficient immunomagnetic separation, Lab Chip, 2011, 11,147
N.M. Dempsey et al., App. Phys. Lett. 90, 092509 (2007)
A. Walther et al., J. Magn. Mag. Mat. 321 (2009) 590
M. Kustov et al., J. App. Phys. 108, 063914 (2010)
F. Dumas-Bouchiat et al., App. Phys. Lett. 96, 102511 (2010)

## Revendications

1. Procédé de capture et de détection d'une molécule dans un échantillon, comprenant les étapes suivantes :
a) le mélange dudit échantillon avec des particules magnétiques, chacune desdites particules étant couplée avec un élément apte à se lier sélectivement à ladite molécule à capturer, de sorte à former au moins un complexe comprenant une particule magnétique, ledit élément et ladite molécule liée audit élément, ces particules magnétiques présentant un diamètre compris entre 5 nm et 500nm,
b) l'immobilisation dudit au moins un complexe sur un support comprenant des microsources de champ magnétique ordonnées, et
c) la détection par fluorescence de la molécule immobilisée,
**caractérisé en ce que** l'organisation spatiale des micros sources de champ magnétique ordonnées définit des zones de gradient de champ magnétique fort et des zones de gradient de champ magnétique faible, **en ce que**
l'étape de détection est mise en oeuvre directement après l'étape d'immobilisation, sans étape intermédiaire de lavage du support, par la détermination de la densité de fluorescence à la surface des micros sources de champ magnétique ordonnées dans les zones de gradient de champ magnétique fort, définissant un signal spécifique, et dans les zones de gradient de champ magnétique faible, définissant un signal non spécifique,
**en ce que** ledit support est un microcanal comprenant dans au moins une de ses parois un réseau de micro-sources magnétiques agencées de manière ordonnée,
et **en ce que** la concentration de particules magnétiques dans le mélange obtenu à l'issue de l'étape a) est supérieure à 10⁶ particules/ml.

2. Procédé selon la revendication 1, **caractérisé en ce que** le mélange obtenu à l'étape a) est injecté à l'entrée du microcanal, circulant dans le microcanal, et **en ce que** les complexes sont progressivement immobilisés sur le fond du microcanal par les micro-sources de champ magnétique lors de l'étape b).

3. Procédé selon la revendication 1 ou 2, **caractérisé en ce que** le microcanal comprend une matrice non magnétique renfermant une pluralité d'agglomérats tridimensionnels ordonnés de micro ou nanoparticules aimantées d'un matériau magnétique dur ou doux, lesdits agglomérats formant les micro-sources de champ magnétique.

4. Procédé selon l'une des revendications 1 à 3, **caractérisé en ce que** la molécule à capturer est un antigène et **en ce que** l'élément apte à se lier à ladite molécule est un anticorps récepteur dudit antigène.

5. Procédé selon la revendication 4, **caractérisé en ce que** l'on ajoute audit mélange un anticorps dit de détection portant un marqueur fluorescent, ledit anticorps étant apte à se lier à l'antigène lié à l'anticorps couplé à la particule magnétique.

6. Procédé selon l'une des revendications 1 à 3, **caractérisé en ce que** la molécule à capturer est un anticorps et **en ce que** l'élément apte à se lier à ladite molécule est un antigène reconnu par ledit anticorps.

## Patentansprüche

1. Verfahren zum Einfangen und Erkennen eines Moleküls in einer Probe, umfassend die folgenden Schritte:
a) Mischen der Probe mit magnetischen Partikeln, wobei jedes der Partikel mit einem Element gekoppelt ist, das in der Lage ist, selektiv an das einzufangende Molekül zu binden, sodass mindestens ein Komplex gebildet wird, der ein magnetisches Partikel, das Element und das an das Element gebundene Molekül umfasst, wobei die magnetischen Partikel einen Durchmesser im Bereich von 5 nm bis 500 nm aufweisen,
b) Immobilisieren des mindestens einen Komplexes auf einer Stütze, die geordnete Magnetfeldmikroquellen umfasst, und
c) Erkennen des immobilisierten Moleküls durch Fluoreszenz,
**dadurch gekennzeichnet, dass** die räumliche Anordnung der geordneten Magnetfeldmikroquellen Zonen mit starkem Magnetfeldgradienten und Zonen mit schwachem Magnetfeldgradienten definiert, dass
der Erkennungsschritt direkt nach dem Immobilisierungsschritt ohne den Zwischenschritt des Waschens der Stütze durchgeführt wird, indem die Fluoreszenzdichte an der Oberfläche der Magnetfeldmikroquellen bestimmt wird, die in den Zonen mit starkem Magnetfeldgradienten angeordnet sind und ein spezifisches Signal definieren, und in den Zonen mit schwachem Magnetfeldgradienten, die ein unspezifisches Signal definieren,
dass die Stütze ein Mikrokanal ist, der in mindestens einer seiner Wände ein Netzwerk von magnetischen Mikroquellen umfasst, die in geordneter Art und Weise angeordnet sind,
und dass die Konzentration der magnetischen Partikel in der nach Schritt a) erhaltenen Mischung mehr als 10⁶ Partikel/ml beträgt.

2. Verfahren nach Anspruch 1, **dadurch gekennzeichnet, dass** die in Schritt a) erhaltene Mischung am Eingang des Mikrokanals injiziert wird, wobei sie im Mikrokanal zirkuliert, und dass die Komplexe durch die Mikromagnetfeldquellen in Schritt b) allmählich auf dem Boden des Mikrokanals immobilisiert werden.

3. Verfahren nach Anspruch 1 oder 2, **dadurch gekennzeichnet, dass** der Mikrokanal eine nichtmagnetische Matrix umfasst, die eine Vielzahl von geordneten dreidimensionalen Agglomeraten von magnetisierten Mikro- oder Nanopartikeln aus einem hart- oder weichmagnetischen Material enthält, wobei die Agglomerate die Mikroquellen des Magnetfelds bilden.

4. Verfahren nach einem der Ansprüche 1 bis 3, **dadurch gekennzeichnet, dass** das einzufangende Molekül ein Antigen ist und dass das Element, das sich an das Molekül binden kann, ein Antikörper ist, der das Antigen rezipiert.

5. Verfahren nach Anspruch 4, **dadurch gekennzeichnet, dass** der Mischung ein sogenannter Erkennungsantikörper zugesetzt wird, der eine fluoreszierende Markierung trägt, wobei der Antikörper in der Lage ist, sich an das Antigen zu binden, das an den an das magnetische Partikel gekoppelten Antikörper gebunden ist.

6. Verfahren nach einem der Ansprüche 1 bis 3, **dadurch gekennzeichnet, dass** das einzufangende Molekül ein Antikörper ist und dass das Element, das sich an das Molekül binden kann, ein Antigen ist, das von dem Antikörper erkannt wird.

## Claims

1. A method for capturing and detecting a molecule in a sample, comprising the following steps:
a) mixing said sample with magnetic particles, each of said particles being coupled with an element capable of binding selectively to said molecule to be captured, so as to form at least one complex comprising a magnetic particle, said element and said molecule bound to said element, these magnetic particles having a diameter comprised between 5 nm and 500 nm,
b) immobilizing said at least one complex on a support comprising ordered magnetic field micro-sources, and
c) detecting the immobilised molecule by fluorescence,
**characterised in that** spatial organization of the ordered magnetic field micro-sources defines strong magnetic field gradient areas and low magnetic field gradient areas, and **in that**
the detection step is implemented directly after the immobilisation step, without any intermediate step of washing the support, by determining the fluorescence density at the surface of the ordered magnetic field micro-sources in the high magnetic field gradient areas, defining a specific signal, and in the low magnetic field gradient areas, defining a non-specific signal,
**in that** said support is a micro-channel comprising in at least one of its walls a network of magnetic micro-sources arranged in an ordered manner,
and **in that** the concentration of magnetic particles in the mixture obtained upon completion of step a) is higher than 10⁶ particles/ml.

2. The method according to claim 1, **characterised in that** the mixture obtained in step a) is injected at the inlet of the microchannel, flowing in the microchannel, and **in that** the complexes are progressively immobilised on the bottom of the microchannel by the magnetic field micro-sources during step b).

3. The method according to claim 1 or 2, **characterised in that** the micro-channel comprises a nonmagnetic matrix embedding a plurality of ordered three-dimensional agglomerates of magnetised micro- or nanoparticles made of a hard or soft magnetic material, said agglomerates forming the magnetic field micro-sources.

4. The method according to one of claims 1 to 3, **characterised in that** the molecule to be captured is an antigen and **in that** the element capable of binding to said molecule is a receptor antibody of said antigen.

5. The method according to claim 4, **characterised in that** a detection antibody carrying a fluorescent marker is added to said mixture, said antibody being capable of binding to the antigen bound to the antibody coupled to the magnetic particle.

6. The method according to one of claims 1 to 3, **characterised in that** the molecule to be captured is an antibody and **in that** the element capable of binding to said molecule is an antigen recognized by said antibody.
